# FASCICULE DE BREVET EUROPEEN

(11) **EP 3 568 088 B1**
(45) Date de publication et mention de la délivrance du brevet: **28.04.2021**
(21) Numéro de dépôt: 18700881.8
(22) Date de dépôt: 11.01.2018
(51) Int. Cl.: A61B 17/12, A61B 17/00

(54) **DISPOSITIF INTRA-ANEVRISMAL**
INTRA-ANEURYSMA-VORRICHTUNG
INTRA-ANEURYSM DEVICE

(30) Priorité: 11.01.2017 FR 1750227
(43) Date de publication de la demande: 20.11.2019
(73) Titulaire: Centre Hospitalier et Universitaire de Montpellier, 34295 Montpellier Cedex (FR); Université de Montpellier, 34090 Montpellier (FR); Satt Axlr, 34090 Montpellier (FR)
(72) Inventeur: COSTALAT, Vincent, 34980 Saint Gely Du Fesc (FR)
(74) Mandataire: Gevers & Orès
(86) Numéro de dépôt international: PCT/EP2018/050698
(87) Numéro de publication internationale: WO 2018/130624

(56) Documents cités:
- WO-A1-2008/151204
- US-A1- 2010 023 105
- US-A1- 2012 296 362

## Description

L'invention a trait au domaine des dispositifs médicaux intra-anévrismaux. De façon plus précise, l'invention a trait aux dispositifs intra-anévrismaux à diversion de flux.

On appelle « anévrisme » une dilatation de la paroi d'une artère. La dilatation provoque une poche anévrismale qui communique avec l'artère grâce à une zone rétrécie connue des praticiens sous le nom « collet ».

La rupture d'un anévrisme, ou plus précisément de la poche anévrismale, peut avoir de lourdes conséquences parmi lesquelles, le décès. Plusieurs facteurs sont à l'origine des anévrismes. Il est à noter que les personnes âgées présentent une prévalence plus importante. Chez ces personnes, le risque de rupture est également plus important notamment en raison de pathologies connexes comme l'hypertension artérielle qui se manifeste sous la forme d'une augmentation de la pression artérielle.

Il s'agit par conséquent d'un problème de santé publique qu'il est nécessaire de traiter. Plusieurs techniques de traitement existent.

L'une des techniques est dite « endovasculaire », celle-ci comprend une étape de traitement de l'anévrisme à l'aide d'un dispositif intra-anévrismal. Ceci peut aussi être un dispositif extra-anévrismal, intra-anévrismal ou les deux. Dans ce cas, l'anévrisme est abordé en suivant le trajet des artères. Un cathéter porteur (d'un dispositif intra-anévrismal) est introduit à travers les artères et dirigé sous contrôle radioscopique. Le dispositif est ensuite déposé dans l'anévrisme. En particulier, le dispositif est déposé dans l'artère porteuse de l'anévrisme dans le cas d'un dispositif extra-anévrismal ou dans l'anévrisme dans le cas d'un dispositif intra-anévrismal. Ceci permet d'isoler l'anévrisme du flux sanguin de l'artère afin d'éviter que la poche anévrismale rompt sous l'effet de la pression sanguine.

Les dispositifs intra-anévrismaux sont connus dans le domaine médical. La demande internationale publiée sous le numéro WO 2006/052322 décrit un dispositif intra-anévrismal qui vise à sécuriser l'implantation des « coils ». L'expression « coils » est un terme anglais désignant une petite spire de métal utilisée pour permettre l'obturation d'un vaisseau. En effet de nombreux dispositifs intra-anévrismaux utilisent des coils pour remplir la poche anévrismale de l'anévrisme à traiter afin de limiter l'afflux de sang vers l'anévrisme. Ces coils peuvent toutefois constituer un danger puisqu'ils peuvent, par inadvertance être positionnés dans l'artère source (gênant la circulation sanguine de ce fait), ils peuvent aussi se déplacer au fil du temps depuis l'anévrisme vers l'artère source (gênant aussi la circulation sanguine). En dépit du fait que ce document WO 2006/052322 décrit un dispositif visant à sécuriser l'insertion des coils, ce dispositif comporte tout de même de nombreux inconvénients, notamment en raison de l'utilisation de coils, tant pour les patients que pour les praticiens. L'utilisation de coils est un traitement délicat par nature pour le praticien.

Dans ce document le dispositif comprend un stent permettant l'ancrage du dispositif dans une artère source, porteuse de l'anévrisme. Le stent est un terme anglais couramment utilisé dans le domaine médical pour désigner un tube de section sensiblement circulaire. L'ancrage se fait par endothélialisation du stent sur la paroi interne de l'artère. Le dispositif comprend une tête, généralement en forme de coupole destinée à loger à l'intérieur de la poche anévrismale. Entre la coupole et le stent, le dispositif comprend une portion intermédiaire amincie au travers de laquelle passe le flux sanguin.

Un inconvénient est que ce dispositif ne peut pas traiter tout type d'anévrisme. En effet, parmi les anévrismes certains sont dits « à collet large ». Un anévrisme à collet large est une situation anatomique difficile qui correspond à une entrée particulièrement ouverte. Avec ce type d'anévrisme, la stabilité des traitements endovasculaire est souvent défaillante. La coupole n'est pas stable et risque de bouger dans la poche anévrismale au fil du temps et même pire sortir de celle-ci, ce qui dans le meilleur des cas rendrait le dispositif inutile et dans le pire des cas pourrait provoquer des complications chez le patient. En effet, un collet large ne fournit pas à la coupole, la zone d'appui (ou de contact) nécessaire pour que la coupole puisse se maintenir dans la poche anévrismale à long terme. Ainsi ce dispositif ne peut traiter efficacement un anévrisme à collet large car celui-ci ne serait pas stable et pourrait être inefficace voire dangereux pour le patient, particulièrement à long terme.

Un autre inconvénient de ce dispositif est que celui-ci peut nécessiter l'utilisation de coils. Il faut par conséquent utiliser un outil supplémentaire pour positionner les coils dans l'anévrisme une fois le dispositif installé sur le patient. Ceci peut rallonger le temps de pose et implique plus d'opérations à réaliser pour le praticien au détriment de la sécurité du patient.

Un autre inconvénient est que ce dispositif présente des défaillances à long terme pour tout type d'anévrisme confondu. En effet son architecture ne lui permet pas un ancrage stable à long terme.

De plus, ce dispositif comporte une partie intermédiaire présentant un caractère thrombogène. Les fils permettant la liaison entre le stent d'ancrage et la demi-coupole occupent une place significative au centre dans l'artère porteuse de l'anévrisme. Cette structure maillée présente alors un emplacement de choix pour une endothélialisation locale et par conséquent une thrombose dans l'artère porteuse.

Un dispositif intra-anévrismal pour le traitement d'un anévrisme a été proposé dans le document US 2012/296362 A1. Toutefois, ce dispositif utilise également des coils.

Un premier objectif est de proposer un dispositif intra-anévrismal qui puisse s'adapter à tout type d'anévrisme et particulièrement aux anévrismes dont le collet est large.

Un deuxième objectif est de proposer un dispositif intra-anévrismal qui ne requiert pas d'ajout de matériel supplémentaire au sein de l'anévrisme (coils).

Un troisième objectif est de proposer un dispositif intra-anévrismal doté d'une grande stabilité quelle que soit la forme de l'anévrisme.

Un quatrième objectif est de proposer un dispositif qui soit stable lorsqu'il est utilisé pour traiter un anévrisme situé au voisinage d'une bifurcation artérielle.

Un cinquième objectif est de proposer un dispositif ayant un ancrage stable à long terme par rapport aux dispositifs existant.

Un sixième objectif est de proposer un dispositif ergonomique pour le praticien.

A cet effet, il est proposé en premier lieu un dispositif intra-anévrismal pour le traitement d'un anévrisme, ce dispositif comprenant :
- un stent de section sensiblement circulaire de diamètre, le stent étant apte et destiné à ancrer le dispositif dans une artère,
- une tête destinée à être insérée dans l'anévrisme, la tête étant apte à réduire significativement un flux sanguin dans l'anévrisme,
- une portion intermédiaire située entre la tête et le stent, et reliant la tête au stent, dans lequel la portion intermédiaire est en forme d'ellipsoïde dont une extrémité distale est reliée à la tête et une extrémité proximale est reliée au stent, et dans lequel un diamètre transversal de la portion intermédiaire est supérieur ou sensiblement supérieur au diamètre du stent, le diamètre transversal étant sensiblement perpendiculaire à un axe longitudinal du dispositif et dans lequel la portion intermédiaire et la tête sont reliées uniquement par un nœud de sorte que la tête soit mobile par rapport à la portion intermédiaire.

Ce dispositif s'adapte aisément à tout type d'anévrisme. Il permet tout particulièrement de traiter les anévrismes à collet large grâce à sa portion intermédiaire en forme d'ellipsoïde et à son diamètre supérieur à celui du stent. Cette géométrie permet aussi avantageusement d'améliorer la stabilité du dispositif intra-anévrismal quelle que soit la forme de l'anévrisme et tout particulièrement d'un anévrisme situé au voisinage d'une bifurcation artérielle. De plus, la partie intermédiaire conjointement avec le stent permettent un ancrage stable à long terme.

Diverses caractéristiques supplémentaires peuvent être prévues seules ou en combinaison :
- la tête est en forme de coupole ;
- la tête comprend autour de son centre, une concavité tournée vers la portion intermédiaire ;
- la portion intermédiaire prolonge le stent de manière continue ;
- la tête est réalisée au moyen d'au moins un fil formant un maillage apte et destiné à dévier en grande partie le flux sanguin ;
- la tête comprend entre huit et deux cent fils et de préférence entre trente-deux et deux cent fils ;
- le stent et/ou la portion intermédiaire sont réalisés au moyen d'au moins un fil formant un maillage apte à permettre le passage d'un flux sanguin au travers de ceux-ci ;
- la forme d'une paroi extérieure de la tête située en regard de la portion intermédiaire est sensiblement la contre-empreinte d'une paroi supérieure de la portion intermédiaire ;
- les fils sont en matériau biocompatible de préférence du nitinol, platine ou titane ;
- au moins un fil est radio-opaque ;
- le dispositif comprend une bague annulaire située sur une extrémité du stent ;
- le dispositif comprend une seconde bague annulaire souple située entre la tête et la portion intermédiaire ;
- la bague annulaire et la seconde bague annulaire sont fabriquées dans un matériau radio-opaque ;
- les bagues sont fixées par sertissage.

D'autres particularités et avantages de l'invention apparaîtront encore dans la description ci-après en relation avec les dessins annexés, donnés à titre d'exemples non limitatifs et dans lesquels :
- la figure 1 est une vue en deux dimensions d'un dispositif de traitement selon l'invention ;
- la figure 2 est une vue en deux dimensions du dispositif de la figure 1, lorsque le dispositif est installé dans un anévrisme situé au voisinage d'une bifurcation artérielle ;
- la figure 3 est une vue en coupe d'une première bague ;
- la figure 4 comprend plusieurs vues en perspective du dispositif selon une variante de réalisation ;
- la figure 5 est une vue en coupe d'une seconde bague ;
- les figures 6a, 6b, 6c et 6d représentent un premier procédé de fabrication du dispositif;
- les figure 7a, 7b et 7c représentent un second procédé de fabrication du dispositif ;
- la figure 8 est une vue en perspective de la tête du dispositif selon une variante de réalisation.

Dans ce qui suit, un anévrisme à collet large est un anévrisme dont le rapport de la hauteur de celui-ci sur la largeur du collet est supérieur à 1,5. La hauteur de l'anévrisme correspond à la distance H qui sépare un point P1 situé au sommet de celui-ci et un point P2 situé à l'entrée de l'anévrisme. La largeur du collet correspond à la distance L qui sépare les bords latéraux de l'entrée de l'anévrisme.

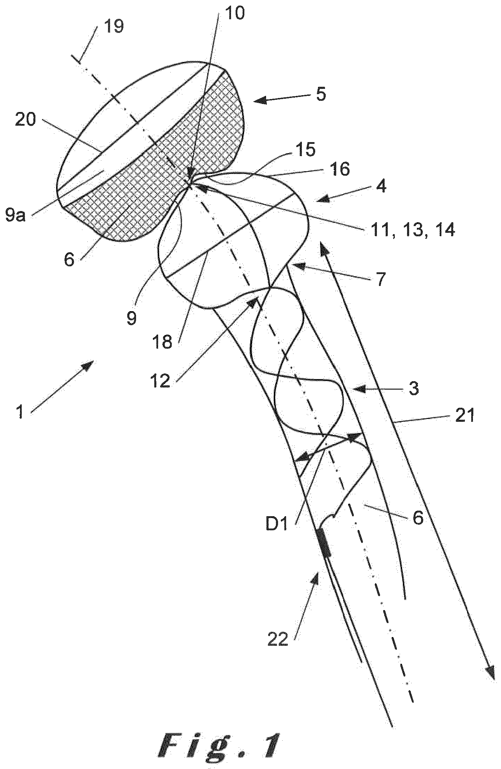

Sur la figure 1 est représenté un dispositif **1** intra-anévrismal. Le dispositif **1** intra-anévrismal est destiné au traitement d'un anévrisme **2.** Ce dispositif **1** est prévu pour un traitement endovasculaire. Il peut être utilisé pour tous les types d'anévrismes **2,** qu'ils soient à collet large ou non. Ce dispositif **1** peut également être utilisé pour traiter les anévrismes **2** quelle que soit la partie du corps touchée.

Dans le mode de réalisation représenté sur la figure 1, le dispositif 1 comprend un stent **3,** une portion **4** intermédiaire et une tête **5** se présentant sous la forme d'une coupole. La portion **4** intermédiaire est située entre le stent **3** et la tête **5.**

Le stent **3,** la portion **4** intermédiaire et la tête **5** sont avantageusement fabriqués à l'aide d'au moins un fil.

Le stent **3,** la portion **4** intermédiaire et la tête **5** sont avantageusement fabriqués à l'aide d'une pluralité de fils **6,** de préférence en nitinol. Le nitinol présente des propriétés mécaniques intéressantes à savoir : la mémoire de forme et son élasticité. De plus, le nitinol est biocompatible et présente un comportement mécanique super élastique.

Avantageusement, au moins un fil est radio-opaque. Ceci permet de visualiser le fil sur un écran, pendant l'intervention permettant également d'observer l'état d'ouverture du dispositif **1.**

Les fils **6** sont agencés les uns par rapport aux autres pour former une maille. Le stent **3** se présente sous la forme d'un tube souple maillé.

La section du stent **3** est avantageusement sensiblement circulaire. A noter toutefois que les caractéristiques du stent **3** font que celui-ci est souple. Ainsi la forme de la section peut varier selon qu'une force est appliquée ou non autour du stent **3.** Le stent peut ainsi s'adapter à la forme de l'artère.

Au repos, c'est-à-dire lorsqu'aucune pression n'est appliquée autour du dispositif 1, la section du stent **3** est par conséquent avantageusement circulaire et présente un diamètre **D1**.

La portion **4** intermédiaire s'étend à partir d'une extrémité **7** supérieure du stent **3.** La portion **4** intermédiaire présente avantageusement un maillage identique à celui du stent **3.** Le portion 4 intermédiaire présente avantageusement un nombre fils identique à celui du stent **3.** Par exemple, le stent **3** et la portion **4** intermédiaire peuvent être chacun formés au moyen de quatre à trente-deux fils **6** de nitinol en fonction de la taille de l'implant. Le nombre de fils **6** peut varier en fonction de la taille de l'implant. Ce nombre de fils permet d'obtenir un maillage large ce qui permet de ne pas bloquer le flux sanguin dans l'artère source, le sang peut ainsi s'écouler librement à travers la portion **4** intermédiaire et à travers le stent **3.** Le risque de formation d'un caillot de sang provoqué par un corps étranger (le dispositif **1**) est par conséquent réduit.

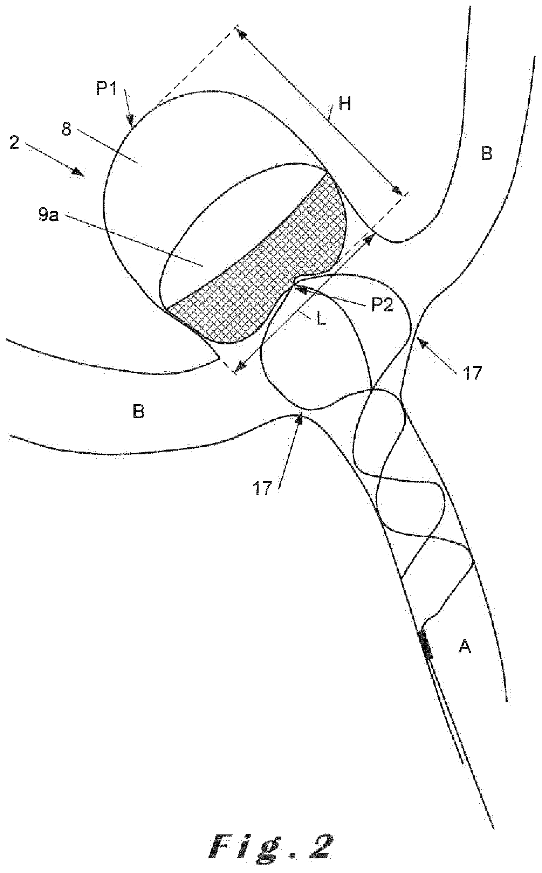

La tête **5** s'étend depuis la portion **4** intermédiaire. Elle présente une forme de coupole concave, dont la concavité **9a** est tournée vers l'extérieur. Une fois le dispositif **1** installé sur un patient, la concavité **9a** fait face à la poche **8** anévrismale ainsi qu'illustré sur la figure 2. A l'instar du stent **3** et de la portion **4** intermédiaire, la maille de la tête **5** est réalisée avec des fils **6** de préférence en nitinol. Néanmoins, à la différence du stent **3** et de la portion **4** intermédiaire, un nombre plus important de fils **6** est utilisé. Alternativement un tressage plus fin peut être réalisé. Entre trente-deux et deux cent fils sont utilisés dans le maillage de la tête **5.** Ainsi la tête **5** est maillée selon un maillage fin, plus fin que celui du stent **3** et de la portion intermédiaire **4,** ce qui permet de réduire fortement l'afflux de sang vers la poche **8** anévrismale sans pour autant totalement l'isoler de manière étanche de l'artère. De cette façon, la poche **8** anévrismale voit sa circulation sanguine ralentir et le sang progressivement stagner dans ladite poche **8** anévrismale pour induire une thrombose rapide. Ceci permet avantageusement de s'affranchir de l'utilisation de coils.

Dans une variante de réalisation la tête 5 comprend entre quatre et trente deux fils à l'instar du stent et de la portion intermédiaire. Dans cette variante de réalisation les fils de la tête forment avec un axe 19 longitudinal du dispositif 1 un angle inférieur que l'angle que forment les fils du stent et de la portion intermédiaire. Par exemple les fils du stent et de la portion intermédiaire forment un angle avec l'axe 19 longitudinal d'environ 50° et les fils de la tête forment un angle avec l'axe 19 longitudinal d'environ 140°. Ceci permet avantageusement d'avoir le même nombre de fils quelle que soit la portion du dispositif tout en s'assurant que la tête remplisse la fonction de diversion du flux sanguin.

La tête **5** comprend une concavité **9** localisée autour d'un centre **10** de la coupole, la concavité **9** étant tournée vers la portion **4** intermédiaire. Cette concavité **9** présente un intérêt tout particulier qui confère à la tête **5** une forme bombée vers l'intérieur de la poche **8** anévrismale de sorte à ce qu'elle ne s'étende pas ou peu dans une artère. En effet, de par ses caractéristiques (maille fine), la tête **5** peut gêner le flux sanguin dans l'artère. La concavité **9** localisée autour du centre **10** de la tête **5** permet d'éviter que cette dernière ne gêne la circulation sanguine dans l'artère en réduisant le bombement intra luminal. En d'autres termes, il s'agit de limiter ou éviter la présence de la tête **5** dans l'artère pour éviter de réduire la section de celle-ci. En effet, une tête **5** qui s'étendrait dans l'artère pourrait provoquer une agrégation plaquettaire rapide ainsi que potentiellement la formation d'un caillot de sang dans l'artère source ou dans la bifurcation artérielle.

La portion **4** intermédiaire est en forme d'ellipsoïde. Dans la présente demande, l'expression « ellipsoïde » n'est pas identique à la définition mathématique universellement connue. L'expression « ellipsoïde » désigne, une forme d'ellipse en trois dimensions. Une ellipse telle que définie mathématiquement a un contour fermé. Pour autant, la portion **4** intermédiaire, même si elle rappelle visuellement une forme ellipsoïdale, ne présente pas un contour fermé notamment au voisinage d'une extrémité **12** proximale. En effet au voisinage de l'extrémité **12** proximale de la portion **4** intermédiaire, celle-ci est ouverte sur le stent **3** de sorte que le volume intérieur que définit la portion intermédiaire communique avec le volume intérieur que définit le stent **3.** Autrement dit, la portion **4** intermédiaire s'inscrit dans la continuité du stent **3,** en prolongeant ce dernier.

Dans une variante de réalisation non représentée sur les figures, la portion **4** intermédiaire et le stent **3** sont reliés par un nœud.

Un nœud est une zone du dispositif où les fils sont tressés, pincés ou tenus par une bague de sorte à réduire significativement le diamètre du dispositif à cet endroit.

Une extrémité **11** distale de la portion **4** intermédiaire est reliée à la tête **5** au moyen d'un nœud **14** au voisinage d'une jonction **13** entre ladite tête **5** et ladite portion **4** intermédiaire. Dans ce qui suit, la liaison entre la portion **4** intermédiaire et la tête **5** sera appelée « liaison nodale ».

La liaison nodale permet avantageusement à la tête **5** d'être mobile par rapport à la portion **4** intermédiaire et globalement par rapport au reste du stent **3.** Ceci permet au dispositif **1** de s'adapter à toutes les anatomies car ainsi, la tête **5** peut se trouver dans un axe différent d'un axe **19** longitudinal du dispositif **1.** Un anévrisme **2** qui ne se trouve pas dans l'axe d'une artère par laquelle passe le dispositif **1** peut par conséquent être traité grâce à la mobilité de la tête **5** rendue possible par la liaison nodale précédemment décrite.

Dans un mode de réalisation préféré représenté sur les figures, la portion **4** intermédiaire présente une forme ellipsoïdale qui se rapproche d'une sphéroïde. A noter qu'une sphère est un cas particulier d'une ellipsoïde.

Ainsi que précédemment expliqué, dans le cas d'un anévrisme **2** à collet large, il n'y pas de zone d'appui pour stabiliser correctement la tête **5** dans la poche **8** anévrismale. La portion **4** intermédiaire dont une forme est ellipsoïdale permet avantageusement de résoudre conjointement avec le stent **3** ce problème de stabilité.

Avantageusement, une paroi **15** extérieure de la tête **5** située en regard de la portion **4** intermédiaire, correspond sensiblement à la contre empreinte d'une paroi **16** supérieure de ladite portion **4** intermédiaire. Ceci permet un contact harmonieux entre la tête **5** et la portion **4** intermédiaire, lorsqu'il y a éventuellement contact entre ces deux éléments. L'expression « harmonieux » désigne le fait que la paroi **15** extérieure présente une forme permettant à la tête **5** de reposer sur la portion **4** intermédiaire, cette dernière peut ainsi éventuellement servir d'assise à la tête **5.**
En référence à la figure 2, le dispositif **1** est inséré dans un anévrisme **2** à collet large. Plus précisément, la tête **5** est insérée dans la poche **8** anévrismale. Lorsque la tête **5** est déployée, ses parois prennent appui sur les parois de la poche **8** anévrismale ce qui confère au dispositif **1** ses conditions de stabilité initiale. La réalisation de la stabilité à long terme du dispositif **1** sera décrite ultérieurement.

Ainsi qu'on peut le voir sur la figure 2, la portion **4** intermédiaire se trouve au niveau d'une bifurcation entre une artère **A** principale et deux artères **B** secondaires. La portion **4** intermédiaire prend appui sur les parois de l'artère A. La portion **4** intermédiaire prend appui au niveau de deux points **17** d'appuis par exemple. Ainsi que pour le stent **3,** la portion **4** intermédiaire sera soumise au phénomène d'endothélialisation qui pourra fixer celle-ci sur la paroi des artères. La portion **4** intermédiaire et le stent **3** assurent ainsi conjointement une stabilité à long terme accrue par rapport aux dispositifs existants.

Avantageusement, un diamètre **18** transversal de la portion **4** intermédiaire est supérieur à un diamètre D1 du stent **3.** Le diamètre **18** transversal de la portion **4** intermédiaire vise la largeur de celle-ci selon une direction transversale à une axe **19** longitudinale du dispositif **1,** autrement dit sensiblement perpendiculaire audit axe **19** longitudinal. Ceci permet à la portion **4** intermédiaire d'avoir une meilleure stabilité.

Dans un mode de réalisation propice pour le traitement d'un anévrisme cérébral, la tête **5** présente une forme de coupole dont le diamètre **20** le plus large est sensiblement compris entre 4 et 13 mm. Le diamètre **D1** du stent **3** est par exemple compris entre 2,5 et 4 mm et sa longueur **21** comprise entre 15 et 25 mm. La portion **4** intermédiaire comprend un diamètre **18** transversal d'environ 4-5 mm. Ces valeurs sont toutefois variables et dépendent principalement de l'endroit où se trouve l'anévrisme à traiter. En effet, un anévrisme situé dans le cerveau n'aura pas les mêmes dimension qu'un anévrisme situé dans une autre partie du corps.

Le dispositif **1** comprend un système de détachement électrolytique qui permet de détacher le dispositif **1** une fois mis en place dans l'anévrisme. Dans le mode de réalisation représenté sur les figures, le système de détachement se situe à une extrémité **22** du stent **3.**

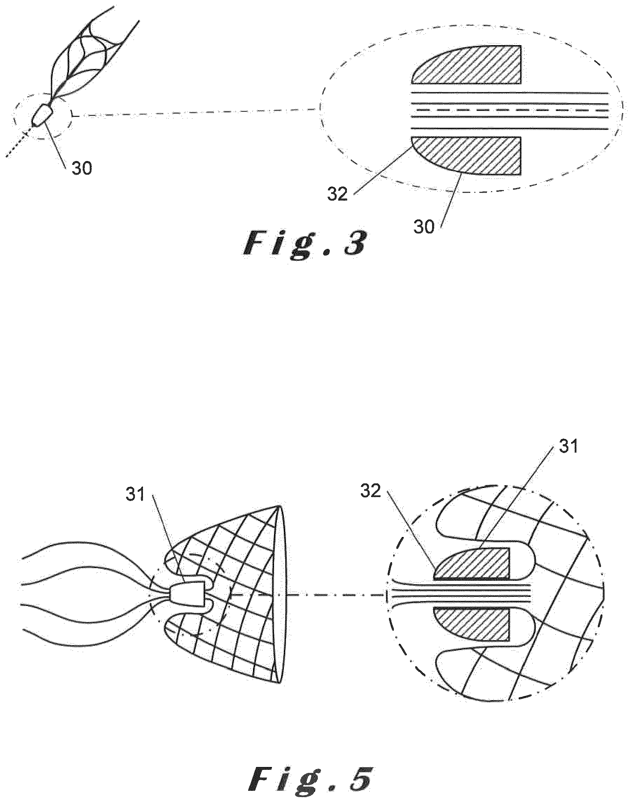

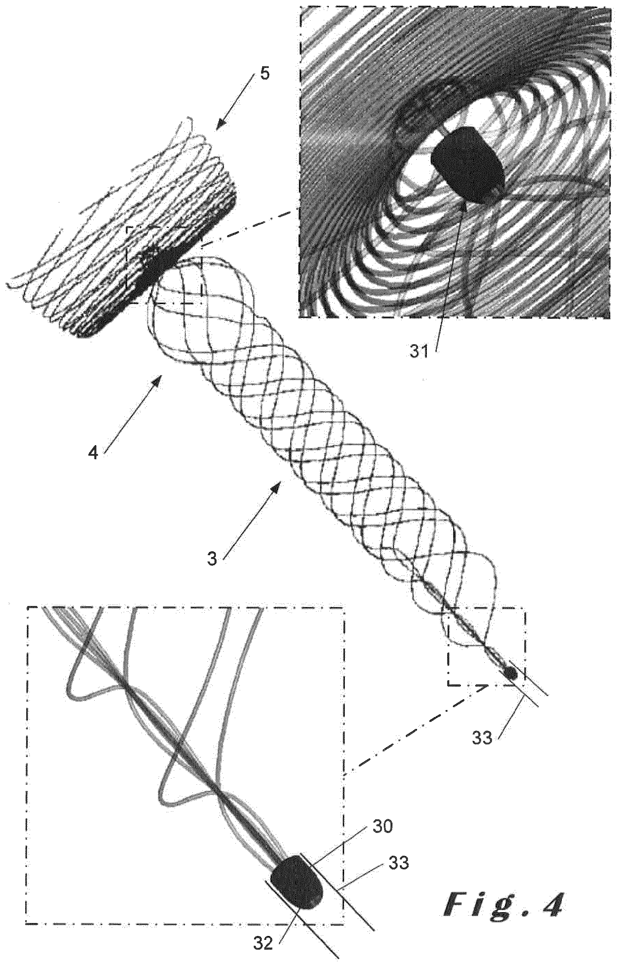

Le dispositif **1** comprend une bague **30** annulaire visible en vue de coupe sur la figure 3 et en perspective sur la figure 4. La bague **30** annulaire est située sur le stent. En particulier la bague **30** annulaire se situe à une extrémité du stent **3.** La bague **30** annulaire est fabriquée dans un matériau radio opaque, tel que le titane ou le platine par exemple. La bague **30** annulaire permet de regrouper l'ensemble des fils constituant le dispositif afin de ravaler l'ensemble desdits fils si besoin, par exemple pour repositionner le dispositif 1.

La bague **30** se présente sous la forme d'un corps annulaire comprenant un bord **32** arrondi. Le bord arrondi est situé au voisinage de l'extrémité du stent **3.** Le bord **32** arrondi permet de faciliter le repliement du dispositif au moyen d'un cathéter **33** de forme cylindrique. Afin de replier le dispositif **1,** des bords du cathéter butent sur le bord **32** arrondi de la bague **30** annulaire, laquelle s'insère au fur et à mesure dans le cathéter. Ceci permet de replier plus facilement le dispositif **1** pour le replacer, le remplacer ou le retirer tant que le phénomène d'endothélialisation ne l'empêche pas.

Avantageusement un diamètre D2 extérieur de la bague **30** annulaire est compris entre 150 et 250 µm et de préférence environ 200 µm. Cette valeur permet avantageusement de faciliter la manutention du dispositif **1** dans un cathéter de 0,5 mm de diamètre. En tout état de cause, le diamètre de la bague est strictement inférieur au diamètre du cathéter de manutention.

Dans une variante de réalisation, le dispositif **1** comporte une seconde bague **31** annulaire identique à la bague **30.** La seconde bague **31** se situe entre la tête **5** et la portion **4** intermédiaire. La seconde bague **31** annulaire sert d'une part de marqueur pour aider le chirurgien à positionner le dispositif dans l'anévrisme et d'autre part permet une meilleure fixation au voisinage du nœud. En tant que marqueur, elle permet au chirurgien de positionner avec précision le dispositif en ce que la seconde bague annulaire est agencée au voisinage du collet de l'anévrisme. La seconde bague **31** annulaire se superpose sur le nœud, tout en gardant une tête **5** parfaitement mobile par rapport à la portion **4** intermédiaire.

La seconde bague 31 permet de positionner avec précision la tête dans l'anévrisme, cette bague étant facilement identifiable par le praticien. En effet, la seconde bague 31 indique au praticien la frontière entre la tête 5 et la portion 4 intermédiaire.

Afin d'éviter que les bagues **30, 31** ne bougent par rapport au dispositif, celles-ci sont solidaires dudit dispositif **1.** Les bagues 30, 31 sont fixées sur le dispositif 1 par sertissage. Le sertissage est un procédé mécanique dans lequel celle-ci est déformée plastiquement pour la solidariser sur le dispositif 1.

Le dispositif 1 décrit ci-dessus présente plusieurs avantages. Ainsi un premier avantage de ce dispositif est qu'il peut s'adapter à tout type d'anévrisme et particulièrement aux anévrismes dont le collet est large. Un deuxième avantage est qu'il peut traiter des anévrismes sans ajout de matériel supplémentaire (coils). Un troisième avantage est que le dispositif est stable quelle que soit la forme de l'anévrisme et plus particulièrement lorsque le collet de l'anévrisme est large. Un quatrième avantage est que le dispositif est également particulièrement stable lorsqu'il est utilisé pour traiter un anévrisme au voisinage d'une bifurcation artérielle. Un cinquième avantage du dispositif est son ancrage stable et efficace à long terme. Un sixième avantage est que le dispositif est ergonomique pour le praticien. En particulier, le dispositif est conçu de manière à être délivré par un micro cathéter de faible diamètre extérieur permettant un accès plus aisé aux anévrismes les plus éloignés et ne nécessitant aucun ajout de matière supplémentaire. Le temps de pose s'en trouve ainsi réduit. Enfin, les marqueurs radio-opaques (fils et/ou bagues) permettent une visualisation en temps réel du positionnement correct du dispositif.

Dans ce qui suit, seront décrits plusieurs procédés de fabrication du dispositif **1.**

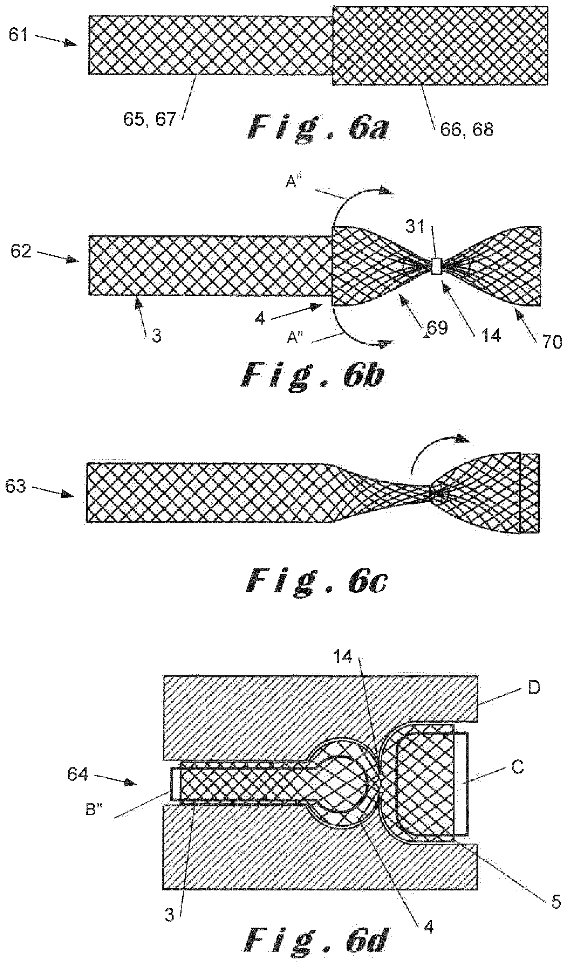

Selon un premier procédé de fabrication du dispositif illustré sur les figures 6a, 6b, 6c et 6d, celui-ci comprend une première étape **61** dans laquelle un premier stent **65** ayant un premier maillage est inséré en partie dans un deuxième stent **66** ayant un second maillage plus fin que le premier maillage, de sorte à former une portion **67** à maille large dont la maille est celle du premier stent **65** et une portion **68** à maille fine dont la maille est la superposition du premier et du deuxième stent 65, 66. Dans une deuxième étape **62,** un nœud **14** est formé dans la portion **68** à maille fine. Le nœud **14** est positionné de telle sorte que de part et d'autre du nœud, il y ait une zone fine **69, 70.** Le nœud **14** est une séparation entre la portion intermédiaire **4** et la tête **5.** Dans une troisième étape **63,** le deuxième stent **68** qui se situe sur la portion intermédiaire **4** et qui constitue avec la premier stent **67** la zone fine **69,** est replié selon des flèches A" en direction de la tête **5** de sorte à ne laisser dans la portion intermédiaire **4** que le premier stent **65.** Ainsi le maillage de la portion **4** intermédiaire est celui du premier stent **65.** Le maillage de la tête **5** comprend trois couches à savoir une première couche du premier stent **65,** une deuxième couche du deuxième stent **66** et une troisième couche du deuxième stent **66** qui correspond à la portion repliée dans la troisième étape **63.** Dans une quatrième étape **64,** un tige **B"** de formage comprenant une extrémité en forme d'ellipsoïde pour former la portion **4** intermédiaire est insérée dans le premier stent **65** jusqu'à venir en butée sur le nœud **14** et un élément **C** de formage dont le galbe est une contre empreinte d'une coupole est inséré dans le deuxième stent **68** en butée jusqu'au nœud **14.** Tous ces éléments sont insérés dans un moule D dont la cavité de moulage définit une portion tubulaire et une portion ellipsoïdale ainsi qu'une portion en forme de coupole. Dans une cinquième étape, le moule est chauffé afin de thermo-fixer le dispositif final.

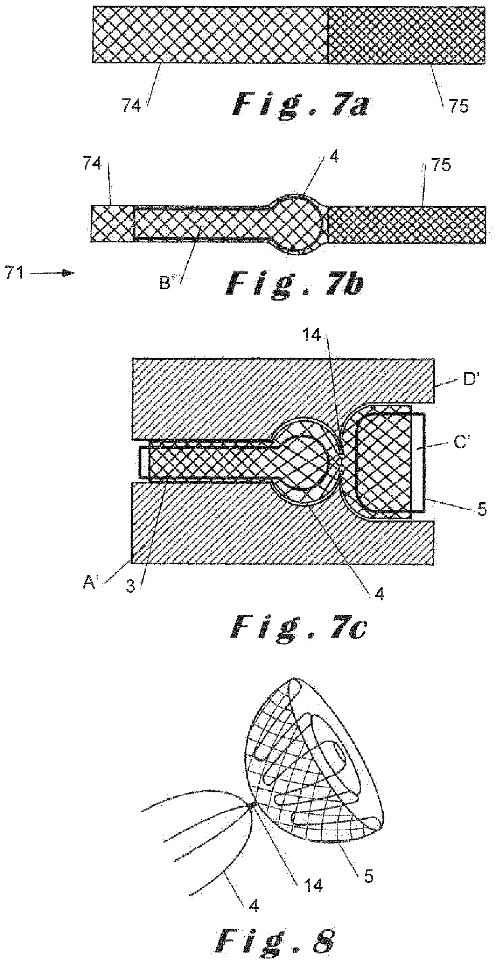

Selon un deuxième procédé de fabrication du dispositif illustré sur les figures 7a, 7b et 7c, celui-ci comprend une première étape dans laquelle une première tige **B'** comportant une extrémité en forme d'ellipsoïde est insérée dans un stent **74.** Le stent **74** comporte une partie **75** à maille large et une partie **75** à maille fine. La première tige **B'** est insérée jusqu'à ce que l'extrémité soit adjacente à la portion **75** à maille fine. Dans une deuxième étape **73,** un élément de formage en forme de coupole est insérée dans la partie **75** à maille fine du stent. Dans cette étape le stent **74** et les éléments **A', C'** sont insérées dans un moule **D'.** L'ensemble est ensuite chauffé dans le moule **D'** pour thermo-fixer le dispositif final. Le nœud **14** est fabriqué dans le moule D' par thermo fixation et grâce à la forme particulière du moule qui permet un rétrécissement localisé du diamètre du dispositif .

Afin d'optimiser la diversion de flux sanguin, la coupole peut présenter une géométrie complexe. La figure 8 présente une géométrie de la coupole permettant de « casser » le flux sanguin. La densité du maillage de la tête rappelant une fleur permet avantageusement une diversion efficace du flux sanguin. La forme de fleur est obtenue en repliant la portion **69** plusieurs fois à l'intérieur de la tête **5.**

Afin de répondre à un besoin industriel, les moules utilisés pour les premier et deuxième procédés peuvent se présenter sous la forme d'un manchon à section variable dans lequel plusieurs dispositifs peuvent être fabriqués simultanément.

Naturellement, pour satisfaire des besoins spécifiques, une personne compétente dans le domaine de l'invention pourra appliquer des modifications dans la description précédente.

## Revendications

1. Dispositif **(1)** intra-anévrismal pour le traitement d'un anévrisme **(2),** ce dispositif **(1)** comprenant :
- un stent **(3)** de section sensiblement circulaire de diamètre (**D1**), le stent **(3)** étant apte et destiné à ancrer le dispositif **(1)** dans une artère,
- une tête **(5)** destinée à être insérée dans l'anévrisme **(2),** la tête **(5)** étant apte à réduire significativement un flux sanguin dans l'anévrisme **(2),**
- une portion **(4)** intermédiaire située entre la tête **(5)** et le stent **(3),** et reliant la tête **(5)** au stent **(3),** la portion **(4)** intermédiaire étant en forme d'ellipsoïde dont une extrémité **(11)** distale est reliée à la tête **(5)** et une extrémité **(12)** proximale est reliée au stent **(3),** un diamètre **(18)** transversal de la portion **(4)** intermédiaire étant supérieur ou sensiblement supérieur au diamètre (**D1**) du stent **(3),** le diamètre **(18)** transversal étant sensiblement perpendiculaire à un axe **(19)** longitudinal du dispositif **(1),**
**caractérisé en ce que** la portion **(4)** intermédiaire et la tête **(5)** sont reliées uniquement par un nœud **(14)** de sorte que la tête **(5)** soit mobile par rapport à la portion **(4)** intermédiaire.

2. Dispositif **(1)** selon la revendication 1 dans lequel la tête **(5)** est en forme de coupole.

3. Dispositif **(1)** selon la revendication 2 dans lequel la tête **(5)** comprend autour de son centre **(10),** une concavité **(9)** tournée vers la portion **(4)** intermédiaire.

4. Dispositif **(1)** selon l'une des revendications précédentes dans lequel la portion **(4)** intermédiaire prolonge le stent **(3)** de manière continue.

5. Dispositif (1) selon l'une des revendications précédentes dans lequel la tête **(5)** est réalisée au moyen d'au moins un fil **(6)** formant un maillage apte et destiné à dévier en grande partie le flux sanguin.

6. Dispositif **(1)** selon la revendication 5 dans lequel la tête **(5)** comprend entre huit et deux cent fils et de préférence entre trente-deux et deux cent fils.

7. Dispositif **(1)** selon l'une des revendications précédentes dans lequel, le stent **(3)** et/ou la portion **(4)** intermédiaire sont réalisés au moyen d'au moins un fil **(6)** formant un maillage apte à permettre le passage d'un flux sanguin au travers de ceux-ci.

8. Dispositif **(1)** selon l'une des revendications précédentes dans lequel la forme d'une paroi **(15)** extérieure de la tête située en regard de la portion **(4)** intermédiaire est sensiblement la contre-empreinte d'une paroi **(16)** supérieure de la portion **(4)** intermédiaire.

9. Dispositif **(1)** selon l'une quelconque des revendications 5 à 7 dans lequel, les fils **(6)** sont en matériau biocompatible de préférence du nitinol, platine ou titane.

10. Dispositif **(1)** selon l'une des revendications 5 à 7 et 9 dans lequel au moins un fil (6) est radio-opaque.

11. Dispositif **(1)** selon l'une quelconque des revendications précédentes dans lequel, celui-ci comprend une bague **(30)** annulaire située sur une extrémité du stent **(3).**

12. Dispositif **(1)** selon l'une quelconque des revendications précédentes dans lequel, celui-ci comprend une seconde bague **(31)** annulaire située entre la tête **(5)** et la portion **(4)** intermédiaire.

13. Dispositif **(1)** selon l'une quelconque des revendications 11 et 12 dans lequel la bague **(30)** annulaire et la seconde bague **(31)** annulaire sont fabriquées dans un matériau radio-opaque.

14. Dispositif **(1)** selon l'une des revendications 11 à 13 dans lequel les bagues **(30, 31)** sont fixées par sertissage.

## Patentansprüche

1. Intra-Aneurysma-Vorrichtung **(1)** zur Behandlung eines Aneurysmas **(2),** wobei diese Vorrichtung umfasst:
- einen Stent **(3)** mit im Wesentlichen kreisförmigem Querschnitt mit Durchmesser **(D1),** wobei der Stent **(3)** dazu geeignet und bestimmt ist, die Vorrichtung **(1)** in einer Arterie zu verankern,
- einen Kopf **(5),** der dazu bestimmt ist, in das Aneurysma **(2)** eingesetzt zu werden, wobei der Kopf **(5)** dazu geeignet ist, einen Blutfluss in dem Aneurysma **(2)** signifikant zu verringern,
- einen Zwischenabschnitt **(4),** der zwischen dem Kopf **(5)** und dem Stent **(3)** liegt und den Kopf **(5)** mit dem Stent **(3)** verbindet, wobei der Zwischenabschnitt **(4)** Ellipsoidform aufweist, von der ein distales Ende **(11)** mit dem Kopf **(5)** verbunden ist, und ein proximales Ende **(12)** mit dem Stent **(3)** verbunden ist, wobei ein Querdurchmesser **(18)** des Zwischenabschnitts **(4)** größer oder im Wesentlichen größer ist als der Durchmesser **(D1)** des Stents **(3),** wobei der Querdurchmesser **(18)** im Wesentlichen zu einer Längsachse **(19)** der Vorrichtung **(1)** senkrecht ist,
**dadurch gekennzeichnet, dass** der Zwischenabschnitt **(4)** und der Kopf **(5)** lediglich durch einen Knoten **(14)** derart verbunden sind, dass der Kopf **(5)** in Bezug auf den Zwischenabschnitt **(4)** beweglich ist.

2. Vorrichtung **(1)** nach Anspruch 1, wobei der Kopf **(5)** eine Kuppelform aufweist.

3. Vorrichtung **(1)** nach Anspruch 2, wobei der Kopf **(5)** um seine Mitte (**10**) eine Konkavität **(9),** die dem Zwischenabschnitt **(4)** zugewandt ist, umfasst.

4. Vorrichtung **(1)** nach einem der vorstehenden Ansprüche, wobei der Zwischenabschnitt **(4)** den Stent **(3)** durchgehend verlängert.

5. Vorrichtung **(1)** nach einem der vorstehenden Ansprüche, wobei der Kopf **(5)** mittels mindestens eines Drahtes **(6),** der eine Vermaschung bildet, die dazu geeignet und bestimmt ist, den Blutfluss zum Großteil abzulenken, realisiert ist.

6. Vorrichtung **(1)** nach Anspruch 5, wobei der Kopf **(5)** zwischen acht und zweihundert Drähten und vorzugsweise zwischen zweiunddreißig und zweihundert Drähten umfasst.

7. Vorrichtung **(1)** nach einem der vorstehenden Ansprüche, wobei der Stent **(3)** und/oder der Zwischenabschnitt **(4)** mittels mindestens eines Drahtes **(6),** der eine Vermaschung bildet, die dazu geeignet ist, das Durchgehen eines Blutflusses durch diese zu erlauben, realisiert sind.

8. Vorrichtung **(1)** nach einem der vorstehenden Ansprüche, wobei die Form einer Außenwand **(15)** des Kopfes, die gegenüber dem Zwischenabschnitt **(4)** liegt, im Wesentlichen die Gegenprägung einer oberen Wand **(16)** des Zwischenabschnitts **(4)** ist.

9. Vorrichtung **(1)** nach einem der Ansprüche 5 bis 7, wobei die Drähte **(6)** aus biokompatiblem Material, vorzugsweise Nitinol, Platin oder Titan, gebildet sind.

10. Vorrichtung **(1)** nach einem der Ansprüche 5 bis 7 und 9, wobei mindestens ein Draht **(6)** strahlenundurchlässig ist.

11. Vorrichtung **(1)** nach einem der vorstehenden Ansprüche, wobei diese einen ringförmigen Ring (**30**), der auf einem Ende des Stents **(3)** liegt, umfasst.

12. Vorrichtung **(1)** nach einem der vorstehenden Ansprüche, wobei diese einen zweiten ringförmigen Ring **(31),** der zwischen dem Kopf **(5)** und dem Zwischenabschnitt **(4)** liegt, umfasst.

13. Vorrichtung **(1)** nach einem der Ansprüche 11 und 12, wobei der ringförmige Ring (**30**) und der zweite ringförmige Ring **(31)** aus einem strahlenundurchlässigen Material gefertigt sind.

14. Vorrichtung **(1)** nach einem der Ansprüche 11 bis 13, wobei die Ringe (**30**, **31)** durch Falzen befestigt sind.

## Claims

1. Intra-aneurysmal device (1) for the treatment of an aneurysm (2), this device (1) comprising:
- a stent (3) with an approximately circular section with diameter (D1), the stent (3) being suitable for and designed to anchor the device (1) in an artery,
- a head (5) designed to be inserted in the aneurysm (2), the head (5) being capable of significantly reducing blood flow in the aneurysm (2),
- an intermediate portion (4) located between the head (5) and the stent (3), and connecting the head (5) to the stent (3), the intermediate portion (4) being in the form of an ellipsoid, of which a distal end (11) is connected to the head (5) and a proximal end (12) is connected to the stent (3), a transverse diameter (18) of the intermediate portion (4) being larger or much larger than the diameter (D1) of the stent (3), the transverse diameter (18) being approximately perpendicular to a longitudinal axis (19) of the device (1),
**characterised in that** the intermediate portion (4) and the head (5) are only connected by a node (14) such that the head (5) is free to move relative to the intermediate portion (4).

2. Device (1) according to any one of the preceding claims, wherein the head (5) is dome-shaped.

3. Device (1) according to claim 2, wherein the head (5) is concave around its centre (10), the concave part (9) facing the intermediate portion (4).

4. Device (1) according to any one of the preceding claims, wherein the intermediate portion (4) continuously prolongs the stent (3).

5. Device (1) according to any one of the preceding claims, wherein the head (5) is composed of at least one wire (6) forming an appropriate mesh and is designed to divert a large proportion of the blood flow.

6. Device (1) according to claim 5, wherein the head (5) comprises between eight and two hundred wires and preferably between thirty-two and two hundred wires.

7. Device (1) according to any one of the preceding claims, wherein the stent (3) and/or the intermediate portion (4) are made using at least one wire (6) forming a mesh that enables blood flow through the stent and the intermediate portion.

8. Device (1) according to any one of the preceding claims, wherein the shape of an external wall (15) of the head facing the intermediate portion (4) is approximately the corresponding shape to an upper wall (16) of the intermediate portion (4).

9. Device (1) according to any one of claims 5 to 7, wherein the wires (6) are made of a biocompatible material, preferably nitinol, platinum or titanium.

10. Device according to one of claims 5 to 7 and 9, wherein at least one wire (6) is radio-opaque.

11. Device (1) according to any one of the preceding claims, wherein the device comprises an annular ring (30) located on one end of the stent (3).

12. Device (1) according to any one of the preceding claims, wherein the device comprises a second annular ring (31) located between the head (5) and the intermediate portion (4).

13. Device (1) according to either claim 11 or 12, wherein the annular ring (30) and the second annular ring (31) are made from a radio-opaque material.

14. Device (1) according to one of claims 11 to 13, wherein the rings (30, 31) are fixed by crimping.
